# EUROPÄISCHE PATENTSCHRIFT

(11) **EP 2 775 299 B1**
(45) Veröffentlichungstag und Bekanntmachung des Hinweises auf die Patenterteilung: **11.05.2016**
(21) Anmeldenummer: 14157700.7
(22) Anmeldetag: 04.03.2014
(51) Int. Cl.: G01N 33/44, B29C 47/92, B29C 45/76, G01N 17/02, G01N 27/07

(54) **Maschine mit einer Vorrichtung zur Erzeugung von Kunststoffschmelze und einer Vorrichtung zum Ermitteln der Korrosivität dieser Kunststoffschmelze, sowie Verfahren zur Korrosivitätsermittlung mittels dieser Maschine**
Machine with a device for producing a plastic melt and a device for determining the corrosivity of this plastic melt, and method of determining the corrosivity with this machine
Machine avec un dispositif de production d'une fonte en matière plastique et un dispositif de détermination de sa corrosivité, et procédé de détermination de la corrosivité avec cette machine

(30) Priorität: 05.03.2013 DE 102013203747
(43) Veröffentlichungstag der Anmeldung: 10.09.2014
(73) Patentinhaber: Fraunhofer-Gesellschaft zur Förderung der angewandten Forschung e.V., 80686 München (DE)
(72) Erfinder: Dr. Steinhoff,, Bernd, 64297 Darmstadt (DE); Dr. Lellinger,, Dirk, 64331 Weiterstadt (DE); Dr. Kothe,, Hans, 65474 Bischofsheim (DE)
(74) Vertreter: Pfenning, Meinig & Partner mbB

(56) Entgegenhaltungen:
- EP-A2- 2 551 659
- US-A- 3 259 840
- US-B1- 8 111 078
- John Beaumont: "'How Will It Mold?' Brand-New Test Method Relates Material, Mold & Machine", Plastics Technology, Issue June 2012, 1. Juni 2012 (2012-06-01), XP055116841, Gefunden im Internet: URL:http://www.ptonline.com/articles/how-w ill-it-mold-brand-new-test-method-relates- material-mold-machine [gefunden am 2014-05-08]

## Beschreibung

Die vorliegende Anmeldung betrifft eine Maschine, enthaltend eine Vorrichtung zur Erzeugung von Kunststoffschmelze sowie eine Vorrichtung zum Ermitteln der Korrosivität einer Kunststoffschmelze. Es betrifft außerdem ein Verfahren zum Messen der Korrosivität von Kunststoffschmelze.

Je nach Zusammensetzung weisen manche Polymerkunststoffe hochgradig korrosive Eigenschaften gegenüber Eisenlegierungen auf. Diese korrosiven Eigenschaften (Korrosivität) wirken sich negativ beispielsweise auf Teile von Kunststoffverarbeitungsmaschinen aus.

Die Korrosivität kann hierbei beispielsweise durch flammhemmende Additive bedingt sein. Diese Additive können beispielsweise unmittelbar mit den Eisenlegierungen reagieren, möglich ist es auch, dass aggressive Chemikalien wie anorganische Säuren erst bei der thermischen Zersetzung mancher Additive bei der Herstellung der Kunststoffschmelze entstehen.

Um ein Kunststoffmaterial zu entwickeln, das einerseits in Bezug z.B. auf Flamrrischutz höchsten Anforderungen genügt und außerdem beispielsweise Kunststoffverarbeitungsmaschinen nicht zu stark angreift, muss hier ein entsprechendes Optimum mittels einer geeigneten Messmethode gefunden werden. Die Methode sollte einerseits reproduzierbare Ergebnisse liefern, andererseits sollte die für die Messung benötigte Materialmenge möglichst klein sein.

Die US 3 259 840 bezieht sich auf eine Vorrichtung und ein Verfahren zum Ermitteln der Korrosivität von feuchter Erde. Diese Messapparatur umfasst ein Gehäuse mit einem Hohlraum zur Füllung mit der Probe sowie zwei Elektoden aus unterschiedlichen Materialien und ein Messelement. Es ist daher die Aufgabe der vorliegenden Erfindung, entsprechende Maschinen bzw. Verfahren zur Verfügung zu stellen, die auf eine kostengünstige und einfache Weise die Korrosivität unterschiedlicher Kunststoffschmelzen ermitteln.

Diese Aufgabe wird durch die Gegenstände der unabhängigen Patentansprüche gelöst. Dies ist zunächst eine Maschine, enthaltend eine Vorrichtung zur Erzeugung von Kunststoffschmelze sowie eine Vorrichtung zum Ermitteln der Korrosivität einer Kunststoffschmelze, enthaltend ein Gehäuse mit einem Hohlraum zur Füllung mit Kunststoffschmelze, eine erste Elektrode aus einem ersten Material und eine zweite Elektrode aus einem zweiten Material, wobei die erste und die zweite Elektrode jeweils eine Kontaktoberfläche zum Hohlraum hin aufweisen und das erste Material ein höheres Standardpotential als das zweite Material aufweist, und die erste und zweite Elektrode außerdem zur Bestimmung eines elektrischen Stroms und/oder einer elektrischen Spannung zwischen den Kontaktoberflächen über ein Messelement miteinander verbindbar sind.

Durch die im Hohlraum befindliche Kunststoffschmelze wird die zweite Elektrode an ihrer Kontaktoberfläche durch Korrosion angegriffen. Der beschriebene Korrosionsvorgang ist messtechnisch auswertbar, wenn die beiden Elektroden sowohl elektrisch leitend über eine elektrisch leitende Verbindung als auch über die dazwischen liegende Schmelze verbunden sind. Der Korrosionsprozess hat zur Folge, dass Elektronen von der Anode (also der Elektrode aus dem "unedleren" Material, d.h. mit dem niedrigeren Standardpotential) zur Kathode (also der Elektrode, das aus einem Material mit einem höheren Standardpotential ist) fließen. Es fließt also bei Ablauf dieses Korrosionsprozesses ein Strom, dessen Stärke über ein Messelement zur Bestimmung des elektrischen Stroms ermittelbar ist. Dies ist wiederum ein Maß für die Korrosivität.

Der Absolutwert des Stroms gibt unter Berücksichtigung der Größe der Kontaktflächen der Elektroden Auskunft über die zu erwartenden Korrosionsraten bzw. Korrosionsgeschwindigkeiten, die von vielen Einflussgrößen abhängen und ohne eine solche Messung nur schwer abschätzbar sind. Alternativ lassen sich auch Aussagen zur Korrosivität aus der Messung anderer elektrischer Größen, z.B. des Potentials zwischen den Elektroden erhalten.

Eine Weiterbildung der Vorrichtung zur Messung der Korrosivität sieht die Installation von zusätzlichen Sensoren vor, vorzugsweise Temperatursensoren, Drucksensoren, Sensoren für optische Spektroskopie, Ultraschallmesstechnik, dielektrische Spektroskopie und/oder Lichtstreuung. Auf diese Weise können sämtliche für die Messung relevanten Parameter normiert werden.

Eine Weiterbildung sieht vor, dass die Vorrichtung zur Messung der Korrosivität eine Öffnung zur Zuführung von Kunststoffschmelze aufweist, beispielsweise zum Anschluss eines Schneckenextruders. Somit kann die erfindungsgemäße Vorrichtung unmittelbar an beliebige Vorrichtungen zur Herstellung von Kunststoffschmelze angegliedert werden, um hier normierte Messungen vorzunehmen.

Eine weitere Weiterbildung sieht vor, dass der mit Kunststoffschmelze befüllbare Hohlraum längsgestreckt ist und senkrecht zu seiner Längsrichtung zumindest bereichsweise einen rechteckigen, runden oder ovalen Querschnitt oder Kombinationen davon aufweist. Der Vorteil ist hierbei, dass diese geometrischen Grundformen die Einbringung von Elektroden mit möglichst großen Kontaktoberflächen, die zum Innenraum des Hohlraums bündig sind, ermöglichen. Außerdem wird bei Standardgeometrien auch die Reinigung des Hohlraums leichter möglich sein.

In einer Weiterbildung ragt die eine oder beide Elektroden in den Hohlraum hinein, so dass die Schmelze über die Elektrodenflächen geschert wird. Der dabei stattfindende mechanische Verschleiß lässt gleichzeitige Rückschlüsse auf die Abrasivität der Schmelze zu.

Eine Weiterbildung sieht vor, dass das erste Material Kupfer, Platin, Palladium, amorpher Kohlenstoff, Rhodium, Iridium, Nickel, Silber, Gold, Eisen oder Legierungen der Vorgenannten ist und/oder das zweite Material Zink oder eine Eisenlegierung, insbesondere Kohlenstoffstahl, ist.

Eine Weiterbildung sieht vor, dass die erste und/oder die zweite Elektrode als Teile eines Einsatzes in das Gehäuse eingelassen sind. Der Vorteil hiervon ist, dass beispielsweise zu Reinigungszwecken oder bei Abnutzung (insbesondere der zweiten Elektrode, also der Elektrode mit dem niedrigeren Standardpotential) ein Auswechseln der Elektrode einfach möglich ist. Beispielsweise kann der Einsatz auch als Schraubeinsatz ausgeführt sein. Hierdurch hält die Befestigung des Einsatzes auch höchsten Drücken stand und ist trotzdem dicht. Außerdem kann auf diese Weise gegebenenfalls die Elektrode "nachjustiert" werden. Vorzugsweise ist die Elektrode elektrisch gegenüber dem Gehäuse durch eine elektrische Isolierung (beispielsweise eine Keramikhülse) isoliert.

Prinzipiell ist es auch möglich, lediglich eine Elektrode als externen Einsatz vorzusehen und bei einem geeigneten Material des Gehäuses (welches beispielsweise selbst aus Kohlenstoffstoffstahl ist) einen definierten Teil des Gehäuses als "andere Elektrode" zu nutzen.

Eine Alternative sieht vor, dass beide Elektroden (d.h. die erste und zweite Elektrode) in einem einzigen Einsatz (elektrisch gegeneinander isoliert) kombiniert sind.

Eine Weiterbildung sieht vor, dass das Gehäuse trennbar ist (beispielsweise zweigeteilt ist) zum Öffnen des Hohlraums und zur besseren Zugänglichkeit zu den Kontaktoberflächen. Hierdurch kann zum einen die Kontaktoberfläche leicht und gründlich gereinigt werden, außerdem ist eine Sichtkontrolle der Oberfläche gut möglich, um beispielsweise Rückschlüsse auf die Abrasivität zu ziehen.

Eine weitere Weiterbildung sieht vor, dass mehrere Paare von ersten und zweiten Elektroden vorgesehen sind. Hierdurch wird es möglich, dass verschiedene Messungen zur gleichen Zeit durchgeführt werden. Beispielsweise ist es möglich, verschiedene Materialien für die zweiten Elektroden vorzusehen, um so für eine ganz bestimmte Kunststoffschmelze unter Verwendung von möglichst wenig Kunststoffmaterial möglichst viele Messwerte zu erhalten.

Ein weiterer Aspekt der Maschine, enthaltend eine Vorrichtung zur Erzeugung von Kunststoffschmelze sowie eine Vorrichtung zum Ermitteln der Korrosivität, wie sie oben beschrieben ist, wird im Folgenden erläutert. Bei den Maschinen bzw. Vorrichtungen zur Erzeugung von Kunststoffschmelze kann es sich beispielsweise um eine Spritzgussmaschine, einen Extruder oder einen Kneter handeln. Hierbei sieht eine vorteilhafte Weiterbildung vor, dass die Maschine zwei Ausgänge für Kunststoffschmelze aufweist, wobei ein erster Ausgang mit der Öffnung der Vorrichtung zum Ermitteln der Korrosivität verbunden ist und der zweite Ausgang der Maschine nicht mit der Vorrichtung zur Ermittlung der Korrosivität verbunden ist. Hierdurch wird es beispielsweise möglich, dass an den zweiten Ausgang, z.B. eine Spritzgussform angeschlossen wird und hier kontinuierlich bestimmte Produkte hergestellt werden. Durch den zusätzlichen ersten Ausgang wird es möglich, im laufenden Betrieb die Korrosivität des jeweiligen Materials zu messen.

Eine vorteilhafte Weiterbildung sieht vor, dass der erste Ausgang getrennt vom zweiten Ausgang verschließbar ist. Somit kann "auf Knopfdruck" eine Messung durchgeführt werden, ansonsten kann die Maschine aber völlig normal betrieben werden.

Die Erfindung betrifft schließlich noch ein Verfahren zum Messen der Korrosivität von Kunststoffschmelze, wobei Kunststoffschmelze in einen Hohlraum eingebracht wird und eine erste Elektrode aus einem ersten Material sowie eine zweite Elektrode aus einem zweiten Material vorgesehen sind und die erste und die zweite Elektrode jeweils eine Kontaktoberfläche zum Hohlraum hin aufweisen, wobei das erste Material ein höheres Standardpotential als das zweite Material aufweist und die erste und die zweite Elektrode außerdem über ein Messelement miteinander verbindbar sind und ein elektrischer Strom und/oder eine elektrische Spannung zwischen erster und zweiter Elektrode ermittelt wird.

Vorzugsweise wird bei Einsatz des Verfahrens weniger als 1000 g Kunststoffschmelze benötigt.

Die Erfindung wird nun anhand mehrerer Figuren erläutert. Es zeigen:
- Fig. 1: eine erfindungsgemäße Maschine mit zwei Ausgängen, wobei der erste Ausgang mit einer Vorrichtung zum Ermitteln der Korrosivität verbunden ist.
- Fig. 2a: einen Querschnitt durch eine erfindungsgemäße Vorrichtung zur Messung der Korrosivität mit einem daran angeschlossenen Schneckenextruder und
- Fig. 2b: die Darstellung einer alternativen Bauform einer erfindungsgemäßen Vorrichtung zum Ermitteln der Korrosivität, wobei hier das Gehäuse im geöffneten Zustand gezeigt ist.

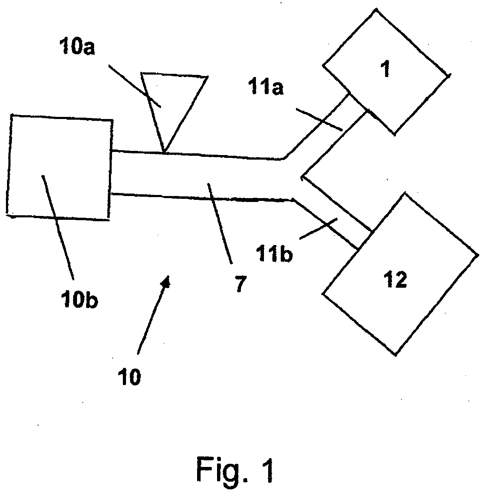

Fig. 1 zeigt (beispielhaft) eine Spritzgussmaschine. Diese weist zunächst eine Vorrichtung zur Erzeugung von Kunststoffschmelze 10 auf, die einen Einfülltrichter zum Einfüllen von Kunststoffgranulat 10a enthält, eine nicht dargestellte Heizeinrichtung sowie einen Antrieb 10b. Am Ende sind ein erster Ausgang 11a und ein zweiter Ausgang 11b angebracht.

Der zweite Ausgang 11b ist mit einer Kunststoffspritzgussform 12 verbindbar, in der beispielsweise Kunststoffwannen etc. herstellbar sind. Der erste Ausgang 11a ist mit einer Öffnung 6 einer Vorrichtung 1 zum Ermitteln der Korrosivität einer Kunststoffschmelze verbunden.

Der Ausgang 11a ist verschließbar, so dass bei einem Verschluss dieses Ausgangs lediglich der Ausgang 11b offen bleibt und ein Fluss von Kunststoffschmelze zu der Vorrichtung 1 unterbunden wird.

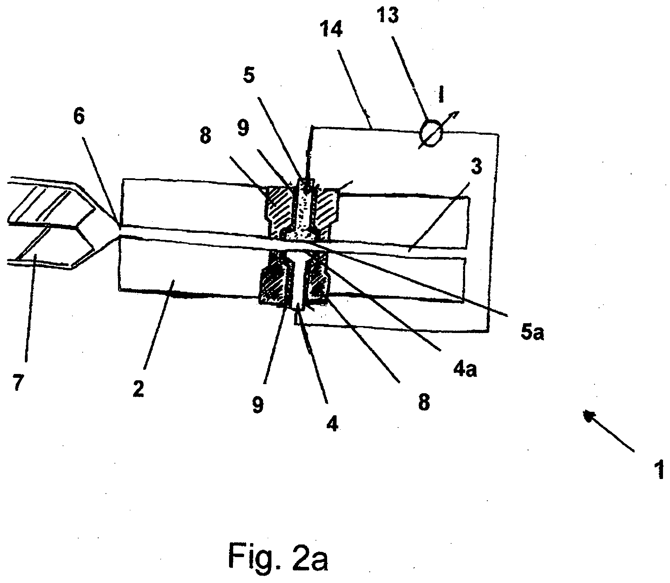

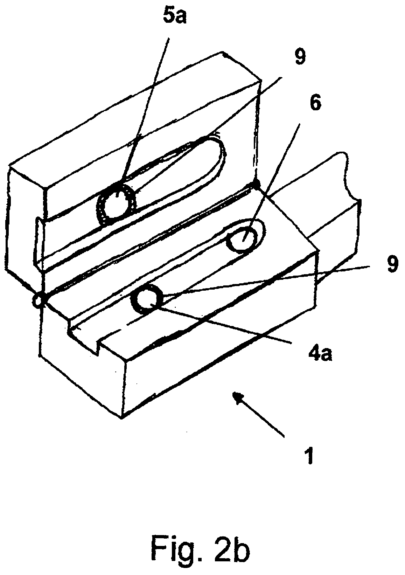

Im Folgenden wird anhand der Fign. 2a und 2b auf Details der Vorrichtung zum Ermitteln der Korrosivität einer Kunststoffschmelze eingegangen.

Fig. 2a zeigt einen Schneckenextruder 7, der an eine Öffnung 6 zur Zuführung von Kunststoffschmelze in die Vorrichtung 1 zur Ermittlung der Korrosivität angeschlossen ist. Die Vorrichtung weist ein Gehäuse 2 auf mit einem mit Kunststoffschmelze befüllbaren Hohlraum 3.

Der Hohlraum kann in Längsrichtung einen rechteckigen, runden oder auch ovalen Querschnitt aufweisen.

Gezeigt sind außerdem zwei Elektroden, eine erste Elektrode 4 sowie eine zweite Elektrode 5. Die erste Elektrode 4 ist aus einem ersten Material und die zweite Elektrode 5 aus einem zweiten Material, wobei das erste Material ein höheres Standardpotential als das zweite Material aufweist.

Die erste Elektrode hat eine Kontaktoberfläche 4a zum Hohlraum hin, die zweite Elektrode hat eine Kontaktoberfläche 5a zum Hohlraum hin. Die Elektroden sind von einer elektrischen Isolierung 9 in Form einer Keramikhülse umgeben, die ebenfalls Teil eines Einsatzes 8 ist, der in das Gehäuse 2 einschraubbar ist. Die Elektroden sind außerdem an ihren dem Hohlraum abgewandten Enden elektrisch miteinander über eine elektrische Leitung 14 und ein Messelement 13 zur Messung von elektrischem Strom verbunden.

Die Kontaktoberfläche 4a bzw. die Kontaktoberfläche 5a sind im Wesentlichen bündig in der Wandung des Hohlraums 3 des Gehäuses 2 der Vorrichtung 1 angebracht. Hierbei bietet es sich an, dass als erstes Material für die erste Elektrode 4 beispielsweise Kupfer, Platin, Palladium oder Legierungen der vorgenannten Metalle verwendet werden und für das zweite Material, also die zweite Elektrode 5, beispielsweise Kohlenstoffstahl. Die in Fig. 2a gezeigte Vorrichtung weist lediglich ein Elektrodenpaar auf. Es ist jedoch auch möglich, entlang der Länge des Hohlraums 3 mehrere Elektrodenpaare einander gegenüberliegend anzuordnen, um so mehrere Messungen (beispielsweise mit unterschiedlichen Elektrodenmaterialien) auf einmal durchführen zu können.

Fig. 2b zeigt den aufgeklappten Zustand einer alternativen Vorrichtung 1. Der Aufbau bezüglich der Elektroden etc. ist hierbei identisch, gut zu sehen ist die senkrecht zur Längsrichtung des Hohlraums 3 rechteckige Querschnittsform des Hohlraums. Bemerkenswert ist der Ort der Zuführung der Kunststoffschmelze (siehe Öffnung 6), der anders als in Fig. 2a ist. Die in Fig. 2b gezeigte Vorrichtung ist zweigeteilt, durch ein Aufklappen des Gehäuses 2 ist der Hohlraum 3 freilegbar, so dass die Kontaktflächen 4a und 5a besonders leicht gereinigt werden können (zusammen mit den übrigen Wänden des Hohlraums), außerdem kann auch leicht eine Sichtprüfung der gereinigten Kontaktoberflächen der Elektroden erfolgen.

Die in den Fign. 1 bis 2b gezeigten Gegenstände eignen sich zur Durchführung eines Verfahrens zum Messen der Korrosivität von Kunststoffschmelze, wobei Kunststoffschmelze in einen Hohlraum (3) eingebracht wird und eine erste Elektrode (4) aus einem ersten Material sowie eine zweite Elektrode (5) aus einem zweiten Material vorgesehen sind und die erste und die zweite Elektrode jeweils eine Kontaktoberfläche zum Hohlraum hin aufweisen, wobei das erste Material ein höheres Standardpotential als das zweite Material aufweist und die erste (4) und die zweite Elektrode (5) außerdem über ein Messelement miteinander verbindbar sind und mittels des Messelements ein elektrischer Strom und/oder eine elektrische Spannung zwischen erster (4) und zweiter (5) Elektrode ermittelt wird.

### Bezugszeichenliste:

- 1: Vorrichtung zum Ermitteln der Korrosivität
- 2: Gehäuse
- 3: Hohlraum
- 4: erste Elektrode
- 4a: Kontaktoberfläche der ersten Elektrode
- 5: zweite Elektrode
- 5a: Kontaktoberfläche der zweiten Elektrode
- 6: Öffnung zur Zuführung von Kunststoffschmelze
- 7: Schneckenextruder
- 8: Einsatz
- 9: Isolierung
- 10: Vorrichtung zur Erzeugung von Kunststoffschmelze
- 10a: Einfülltrichter
- 10b: Antrieb
- 11a: erster Ausgang
- 11b: zweiter Ausgang
- 12: Kunststoffspritzgussform
- 13: Messelement
- 14: elektrische Leitung

## Patentansprüche

1. Maschine, enthaltend eine Vorrichtung zur Erzeugung von Kunststoffschmelze sowie eine Vorrichtung (1) zum Ermitteln der Korrosivität dieser Kunststoffschmelze, enthaltend
• ein Messelement (13),
• ein Gehäuse (2) mit einem Hohlraum (3) zur Füllung mit Kunststoffschmelze,
• eine erste Elektrode (4) aus einem ersten Material und eine zweite Elektrode (5) aus einem zweiten Material, wobei die erste und die zweite Elektrode jeweils eine Kontaktoberfläche (4a, 5a) zum Hohlraum hin aufweisen, wobei
• das erste Material ein höheres Standardpotential als das zweite Material aufweist, und
• die erste (4) und zweite Elektrode (5) außerdem zur Bestimmung eines elektrischen Stroms und/oder einer elektrischen Spannung zwischen den Kontaktoberflächen (4a, 5a) über das Messelement (13) miteinander verbunden sind.

2. Maschine nach Anspruch 1, **dadurch gekennzeichnet, dass** diese eine Öffnung (6) zur Zuführung von Kunststoffschmelze aufweist, vorzugsweise zum Anschluss eines Schneckenextruders.

3. Maschine nach einem der vorhergehenden Ansprüche, **dadurch gekennzeichnet, dass** der Hohlraum (3) zumindest bereichsweise längsgestreckt ist und senkrecht zur Längsrichtung des Hohlraums (3) vorzugsweise einen rechteckigen, runden oder ovalen Querschnitt oder Kombinationen davon aufweist.

4. Maschine nach einem der vorhergehenden Ansprüche, **dadurch gekennzeichnet, dass** das erste Material Kupfer, Platin, Palladium, amorpher Kohlenstoff, Rhodium, Iridium, Nickel, Silber, Gold, Eisen oder Legierungen der Vorgenannten ist und/oder das zweite Material Zink oder eine Eisenlegierung, insbesondere Kohlenstoffstahl, ist.

5. Maschine nach einem der vorhergehenden Ansprüche, **dadurch gekennzeichnet, dass** die erste und/oder die zweite Elektrode (4, 5) als Teile eines Einsatzes (8) in das Gehäuse eingelassen sind.

6. Maschine nach Anspruch 5, **dadurch gekennzeichnet, dass** der Einsatz eine elektrische Isolierung (9) der Elektrode gegenüber dem Gehäuse aufweist.

7. Maschine nach einem der vorhergehenden Ansprüche, **dadurch gekennzeichnet, dass** das Gehäuse (2) trennbar ist zum Öffnen des Hohlraums (3) und zur besseren Zugänglichkeit zu den Kontaktoberflächen (4a, 5a).

8. Maschine nach einem der vorhergehenden Ansprüche, **dadurch gekennzeichnet, dass** mehrere Paare von ersten und zweiten Elektroden (4, 5) vorgesehen sind.

9. Maschine nach einem der vorhergehenden Ansprüche, enthaltend zwei Ausgänge für Kunststoffschmelze, wobei ein erster Ausgang (11a) mit der Öffnung (6) der Vorrichtung zum Ermitteln der Korrosivität verbunden ist und der zweite Ausgang (11b) nicht mit der Vorrichtung zur Ermittlung der Korrosionsstärke verbunden ist.

10. Maschine nach Anspruch 9, **dadurch gekennzeichnet, dass** der erste Ausgang (11a) getrennt vom zweiten Ausgang (11b) verschließbar ist.

11. Verfahren zum Messen der Korrosivität von Kunststoffschmelze mittels einer Vorrichtung nach einem der vorher gehenden Ansprüche, wobei Kunststoffschmelze in den Hohlraum (3) eingebracht wird und ein elektrischer Strom und/oder eine elektrische Spannung zwischen erster (4) und zweiter (5) Elektrode ermittelt wird.

## Claims

1. Machine, comprising a device for generating plastic melt and a device (1) for ascertaining the corrosiveness of this plastic melt, comprising
• a measuring element (13),
• a housing (2) having a cavity (3) to be filled with plastic melt,
• a first electrode (4) made of a first material and a second electrode (5) made of a second material, the first electrode and the second electrode each having a contact surface (4a, 5a) towards the cavity,
• the first material having a higher standard potential than the second material, and
• the first electrode (4) and the second electrode (5) additionally being connectable to each other via the measuring element (13) for determining an electric current and/or an electric voltage between the contact surfaces (4a, 5a) .

2. Machine according to claim 1, **characterised in that** this machine has an opening (6) for feeding plastic melt, preferably for connecting a screw-type extruder.

3. Machine according to 'one of the preceding claims, **characterised in that** the cavity (3) is elongated at least in some regions and, perpendicularly to the longitudinal direction of the cavity (3), preferably has a rectangular, round or oval cross-section, or combinations thereof.

4. Machine according to one of the preceding claims, **characterised in that** the first material is copper, platinum, palladium, amorphous carbon, rhodium, iridium, nickel, silver, gold, iron, or alloys of these, and/or the second material is zinc or an iron alloy, in particular carbon steel.

5. Machine according to one of the preceding claims, **characterised in that** the first and/or the second electrode (4, 5) are recessed into the housing as parts of an insert (8).

6. Machine according to claim 5, **characterised in that** the insert comprises electrical insulation (9) of the electrode with respect to the housing.

7. Machine according to one of the preceding claims, **characterised in that** the housing (2) can be separated to open the cavity (3) and to have better access to the contact surfaces (4a, 5a).

8. Machine according to one of the preceding claims, **characterised in that** a plurality of pairs of first and second electrodes (4, 5) are provided.

9. Machine according to one of the preceding claims, comprising two outlets for plastic melt, a first outlet (11a) being connected to the opening (6) of the device for ascertaining the orrosiveness and the second outlet (11b) not being connected to the device for ascertaining the corrosiveness.

10. Machine according to claim 9, **characterised in that** the first outlet (11a) can be closed separately from the second outlet (11b).

11. Method for measuring the corrosiveness of plastic melt by means of a device according to one of the preceding claims, wherein plastic melt is introduced into the cavity (3) and an electric current and/or an electric voltage between the first electrode (4) and the second electrode (5) is ascertained.

## Revendications

1. Machine comprenant un dispositif pour la production d'une masse fondue de matière plastique ainsi qu'un dispositif (1) pour la détermination de la corrosivité de cette masse fondue de matière plastique, comprenant
- un élément de mesure (13),
- un boîtier (2) avec un espace creux (3) destiné à être remplie de masse fondue de matière plastique,
- une première électrode (4) constituée d'un premier matériau et une deuxième électrode (5) constituée d'un deuxième matériau, la première et la deuxième électrode présentant chacune une surface de contact (4a, 5a) orientée vers l'espace creux,
- le premier matériau présentant un potentiel standard plus élevé sur le deuxième matériau, et
- la première électrode (4) et la deuxième électrode (5) étant en outre reliées entre elles par l'intermédiaire de l'élément de mesure (13) pour la détermination d'un courant électrique et/ou d'une tension électrique entre les surfaces de contact (4a, 5a).

2. Machine selon la revendication 1, **caractérisée en ce que** celle-ci comprend une ouverture (6) pour l'introduction d'une masse fondue de matière plastique, de préférence pour le raccordement d'une extrudeuse à vis sans fin.

3. Machine selon l'une des revendications précédentes, **caractérisée en ce que** l'espace creux (3) est étiré au moins partiellement dans la direction longitudinale et, perpendiculairement à la direction longitudinale de l'espace creux (3), de préférence présente une section transversale rectangulaire, ronde ou ovale ou des combinaisons de ceux-ci.

4. Machine selon l'une des revendications précédentes, **caractérisée en ce que** le premier matériau est du cuivre, du platine, du palladium, du carbone amorphe, du rhodium, de l'iridium, du nickel, de l'argent, de l'or, du fer ou des alliages de ceux-ci et/ou le deuxième matériau est du zinc ou un alliage de fer, plus particulièrement un acier au carbone.

5. Machine selon l'une des revendications précédentes, **caractérisée en ce que** la première et/ou la deuxième électrode (4, 5) sont introduites dans le boîtier en tant que partie d'un insert (8).

6. Machine selon la revendication 5, **caractérisée en ce que** l'insert comprend une isolation électrique (9) de l'électrode par rapport au boîtier.

7. Machine selon l'une des revendications précédentes, **caractérisée en ce que** le boîtier (2) est divisible pour l'ouverture de l'espace creux (3) et pour faciliter l'accès aux surfaces de contact (4a, 5a).

8. Machine selon l'une des revendications précédentes, **caractérisée en ce que** plusieurs paires de premières et de deuxièmes électrodes (4, 5) sont prévues.

9. Machine selon l'une des revendications précédentes, comprenant deux sorties pour la masse fondue de matière plastique, une première sortie (11a) étant reliée avec l'ouverture (6) du dispositif de détermination de la corrosivité et la deuxième sortie (11b) n'étant pas reliée avec le dispositif de détermination de la corrosivité.

10. Machine selon la revendication 9, **caractérisée en ce que** la première sortie (11 a) peut être fermée séparément de la deuxième sortie (11 b).

11. Procédé de mesure dé la corrosivité au moyen d'un dispositif selon l'une des revendications précédentes, de la masse fondue de matière plastique étant introduite dans l'espace creux (3) et un courant électrique et/ou une tension électrique étant déterminée entre une première électrode (4) et une deuxième électrode (5).
